# FASCICULE DE BREVET EUROPEEN

(11) **EP 3 250 241 B1**
(45) Date de publication et mention de la délivrance du brevet: **30.06.2021**
(21) Numéro de dépôt: 16703585.6
(22) Date de dépôt: 20.01.2016
(51) Int. Cl.: A61L 2/08, B65B 55/08

(54) **DISPOSITIF ET PROCÉDE DE STÉRILISATION DE RÉCIPIENTS EN MATIÈRE THERMOPLASTIQUE AU MOYEN D'UN FAISCEAU D'ÉLECTRONS PULSÉ ET D'UN RÉFLECTEUR MOBILE**
VORRICHTUNG UND VERFAHREN ZUM STERILISIEREN VON THERMOPLASTISCHEN BEHÄLTERN MITTELS EINES GEPULSTEN ELEKTRONENSTRAHLS UND EINES MOBILEN REFLEKTORS
DEVICE AND METHOD FOR STERILIZING THERMOPLASTIC CONTAINERS USING A PULSED ELECTRON BEAM AND A MOBILE REFLECTOR

(30) Priorité: 28.01.2015 FR 1550638
(43) Date de publication de la demande: 06.12.2017
(73) Titulaire: Sidel Participations, 76930 Octeville-sur-Mer (FR)
(72) Inventeur: BIANCHINI, Cédric, 76930 Octeville-sur-Mer (FR); FEUILLOLEY, Guy, 76930 Octeville-sur-Mer (FR)
(74) Mandataire: Sidel Group
(86) Numéro de dépôt international: PCT/FR2016/050099
(87) Numéro de publication internationale: WO 2016/120544

(56) Documents cités:
- EP-A1- 2 213 578
- FR-A1- 2 140 393
- FR-A1- 2 140 393
- US-A1- 2003 164 285
- US-B2- 8 728 393

## Description

La présente invention concerne un dispositif et un procédé de stérilisation de récipients en matière thermoplastique au moyen d'un faisceau d'électrons pulsé.

On connaît de l'état de la technique différents procédés de stérilisation pour stériliser au moins l'intérieur d'une préforme et/ou d'un récipient en matière thermoplastique.

La fabrication d'un récipient en matière thermoplastique est obtenue à partir d'une préforme chaude, généralement préalablement conditionnée thermiquement dans un four d'une installation de fabrication de récipients avant d'être introduite dans un moule pour y être transformée par soufflage au moyen d'au moins un fluide sous pression, avec ou sans étirage.

On fabrique ainsi différents types de récipients (bouteilles, flacons, pots, etc.) formant des corps creux qui sont notamment, mais non exclusivement, destinés à être utilisés pour le conditionnement de produits dans l'industrie agro-alimentaire.

Dans le domaine de la fabrication de récipients pour l'industrie agro-alimentaire, on recherche par tous moyens à réduire les risques de contaminations microbiologiques des récipients par des agents pathogènes, soit des micro-organismes.

C'est la raison pour laquelle, la Demanderesse a déjà proposé de mettre en œuvre différentes actions pour éliminer des agents pathogènes, tels que les germes (bactéries, moisissures, etc.), qui sont susceptibles d'affecter le produit contenu dans de tels récipients.

Les documents de l'état de la technique cités ci-après et auxquels on se reportera pour de plus amples détails, illustrent à titre d'exemples non limitatifs de telles actions.

On peut en particulier distinguer d'une part les actions visant à détruire les micro-organismes pour stériliser au moins l'intérieur du récipient et, d'autre part, les actions visant plus généralement à prévenir la contamination des récipients par de tels micro-organismes.

Le document FR-2.915.127 décrit une installation de fabrication de récipients comportant une enceinte de protection délimitant une zone à l'intérieur de laquelle est agencée une machine de moulage de récipients de type souffleuse qui est alimentée par des moyens de transfert en préformes préalablement conditionnées thermiquement dans un four.

Selon les enseignements de ce document, l'installation comporte un système d'insufflation d'air filtré à l'intérieur de l'enceinte pour y établir notamment une surpression de manière à limiter les risques de contamination tant des préformes en sortie du four que des récipients fabriqués.

Le document WO-03/084818 décrit par exemple un traitement de décontamination par irradiation du col de préformes par un rayonnement de type ultraviolet (UV), avant l'introduction des préformes dans le four.

Le document EP-2.094.312 décrit par exemple un traitement par irradiation avec un rayonnement ultraviolet (UV) mis en œuvre de manière particulière dans un four pour décontaminer au moins la surface externe de la préforme en cours de conditionnement thermique.

Le document WO-2006/136498 décrit par exemple un traitement de décontamination d'une préforme consistant à déposer par condensation un film de buée sensiblement uniforme d'un agent stérilisant sur la paroi interne de la préforme.

La décontamination de la préforme y est réalisée au moyen d'un dispositif de traitement intervenant avant l'introduction de la préforme dans le four.

Un tel traitement est destiné à détruire les agents pathogènes ou micro-organismes pour décontaminer au moins l'intérieur de la préforme correspondant à la surface interne dite « alimentaire » du récipient en devenir, c'est-à-dire celle qui sera, après remplissage, en contact direct avec le produit.

On rappelle que la quantité de micro-organismes est susceptible d'être dénombrée par comptage après notamment des opérations de lavage, de filtration et de mise en culture.

On détermine ainsi une réduction logarithmique du nombre de micro-organismes par exemple dite de l'ordre de 3Log (ou encore 3D) équivalent à 1000 unités (10³).

Un tel traitement de décontamination par condensation, dit par « voie chimique », donne satisfaction puisque des degrés de décontamination jusqu'à 6Log sont obtenus.

Cependant, on recherche des solutions alternatives permettant de ne pas utiliser d'agent stérilisant, comme le peroxyde d'hydrogène (H₂O₂), et ce afin de trouver des solutions qui soient plus respectueuses de l'environnement, mais sans toutefois sacrifier pour autant au résultat obtenu pour la décontamination.

L'utilisation d'agent stérilisant, tel que le peroxyde d'hydrogène, nécessite la mise en œuvre d'un ensemble de moyens particuliers pour répondre notamment à des obligations réglementaires visant à protéger les personnels exposés et plus généralement l'environnement (gestion des effluents, etc.), cela contribuant à en augmenter les coûts d'exploitation.

Bien entendu, les différents exemples d'actions précitées sont avantageusement susceptibles d'être mis en œuvre en combinaison dans une même installation pour traiter les différentes surfaces d'une préforme et plus généralement pour réduire de manière drastique les risques de contamination.

Les récipients en matière thermoplastique, telle que le PET (PolyEthylène Terephtalate), dont il est question ici sont notamment mais non exclusivement des bouteilles.

Un tel récipient creux est délimité dans son ensemble par une paroi et comporte un col délimitant radialement une ouverture et se prolongeant par un corps fermé axialement par un fond.

Pour la stérilisation de l'intérieur de ce type de récipients en matière thermoplastique, l'un des problèmes rencontrés demeure l'accessibilité limitée à la surface intérieure du récipient qu'offre l'ouverture du col qui présente généralement un diamètre réduit.

L'une des solutions alternatives consiste à utiliser un rayonnement ionisant formé par un faisceau d'électrons qui est utilisé pour irradier la surface à stériliser.

L'agencement d'un émetteur du faisceau d'électrons à l'extérieur du récipient permet de s'affranchir de ce problème d'accessibilité limitée, les électrons du faisceau émis par l'émetteur traversent, de l'extérieur vers l'intérieur, radialement la paroi du corps et du col dudit récipient pour irradier l'intérieur du récipient à stériliser.

Toutefois, lorsque le faisceau d'électrons de type continu (en anglais « *continuous e-beam* ») utilisé est émis par un émetteur dit à « haute énergie », c'est à dire généralement avec une énergie supérieure à 500 KeV et par exemple de l'ordre du MeV, on constate alors que les électrons d'un tel faisceau continu provoquent des modifications de la matière thermoplastique avec laquelle lesdits électrons interagissent en traversant la paroi du récipient.

Or de telles modifications altèrent les propriétés de la matière thermoplastique du récipient et sont de nature à en compromettre l'utilisation ultérieure comme emballage.

Afin de limiter les interactions entre le faisceau d'électrons continu et la matière thermoplastique, l'utilisation d'un émetteur à basse énergie (inférieure à 500 KeV) a été envisagée.

Cependant, le moindre niveau d'énergie des électrons du faisceau continu se traduit par une stérilisation insuffisante à partir du moment où le faisceau doit traverser la paroi du récipient, du corps comme du col, pour parvenir à en irradier la surface intérieure.

Le degré de stérilisation souhaité ne peut donc être obtenu qu'en augmentant la durée d'irradiation pour compenser la faible pénétration du faisceau d'électrons continu à basse énergie mais les durées nécessaires au traitement d'un récipient sont alors incompatibles avec les cadences de fabrication.

De plus, des problèmes d'interactions entre le faisceau d'électrons continu et la matière thermoplastique demeurent et l'altération de la matière thermoplastique est d'autant plus importante que la durée d'irradiation est longue.

Selon une solution connue du document US-8,728,393, une partie des problèmes peut être résolue en introduisant le faisceau d'électrons continu par l'ouverture du col, directement à l'intérieur, sans traverser la paroi.

Compte tenu des diamètres de col d'une préforme (ou d'un récipient), l'émetteur demeure cependant à l'extérieur de la préforme et le faisceau d'électrons continu doit être amené, guidé jusqu'à l'intérieur pour pouvoir réaliser l'irradiation.

Une telle solution est particulièrement complexe à mettre en œuvre pour être exploitée industriellement et pour parvenir à une irradiation de l'ensemble de la surface interne, seule à même de garantir une stérilisation fiable.

En procédant à l'irradiation de l'intérieur de la préforme selon le document US-8,728,393 et non pas à celle du récipient obtenu à partir d'une telle préforme, il existe également un risque de contamination de la préforme ou du récipient postérieurement à la stérilisation de sorte que, à tout le moins, des mesures préventives drastiques doivent ensuite être mises en œuvre pour limiter tout risque de contamination postérieurement à la stérilisation par irradiation.

Lorsque des moyens de guidage du faisceau d'électrons sont introduits axialement à l'intérieur de la préforme pour procéder à la stérilisation, il existe alors un risque de contamination de l'intérieur de la préforme, récipient en devenir.

En effet, de tels moyens de guidage ne sont pas stériles et peuvent être le vecteur d'une contamination par des micro-organismes, en particulier une contamination du buvant c'est-à-dire du bord circonférentiel du col délimitant l'ouverture de la préforme ou du récipient.

"Le document FR2140393 décrit un procédé de stérilisation d'objets. Les radiations émises parviennent à l'intérieur de l'objet en passant par une ouverture de l'objet. Cela ne convient pas pour strériliser des bouteilles.

Le document US2003 164285 décrit un appareil mobile pour steriliser notamment du courrier, avec des radiations de faisceaux d'électrons et de rayons X.

Le document EP2213578 décrit une appareil et un procédé de stérilisation d'une bouteille par un faisceau d'électron irradiant la boutielle depuis l'extérieur."

Bien que pour l'essentiel les solutions connues de l'état de la technique utilisent un faisceau d'électrons de type continu, le document FR-2.861.215 divulgue également l'utilisation d'un faisceau d'électrons à basse énergie, de type pulsé, pour la stérilisation d'emballages tels que des bouteilles.

Tel que décrit dans ce document, le faisceau d'électrons de type pulsé est notamment obtenu en n'appliquant pas de manière permanente, mais seulement pendant un temps donné, la tension provoquant l'accélération des électrons du faisceau.

Avec un émetteur comme le canon à électrons à anode focalisante du document FR-2.861.215, il est indiqué à titre d'exemple que la tension est appliquée pendant 2 µs (microseconde) avec une fréquence de 500 Hz, soit une émission toutes les 2 ms (milliseconde), et un courant ayant une intensité de 10 A.

L'utilisation d'un canon à électrons à anode focalisante selon le document FR-2.861.215 ou un émetteur analogue ne donne toutefois pas satisfaction pour stériliser l'intérieur de récipients en matière thermoplastique.

En effet, la durée de traitement nécessaire pour irradier avec une quantité suffisante d'électrons la surface à stériliser, c'est-à-dire pour obtenir une dose létale, est trop importante et n'est par conséquent pas compatible avec les cadences actuelles de production de récipients qui, dans le cas de bouteilles en PET atteignent par exemple 50.000 à 60.000 bouteilles par heure.

Les problèmes d'altération de la matière thermoplastique subsistent également avec un tel faisceau d'électrons pulsé qui ne permettent pas plus d'en envisager l'application industrielle.

Les différentes solutions alternatives connues de l'état de la technique et qui viennent d'être décrites ne donnent par conséquent pas satisfaction pour stériliser au moins l'intérieur de récipients en matière thermoplastique, en particulier des bouteilles en PET.

Le but de la présente invention est notamment de résoudre au moins une partie des inconvénients de l'état de la technique et de proposer une solution permettant, sans en dégrader la matière constitutive, de stériliser de manière fiable et rapide l'intérieur d'un récipient en matière thermoplastique.

Dans ce but, l'invention propose un procédé de stérilisation de récipients selon la revendication 1.

Avantageusement, le faisceau d'électrons de type pulsé selon l'invention est discret, formé par une succession d'impulsions présentant une intensité très élevée de l'ordre du kilo-Ampère (kA) et avec une durée d'émission particulièrement brève de l'ordre de la nanoseconde (ns).

Grâce à un faisceau d'électrons pulsé présentant conformément à l'invention une durée d'émission inférieure à 100 ns et une intensité supérieure à 1 kA, la stérilisation de l'intérieur du récipient est obtenue, avec non seulement un temps de traitement compatible avec les cadences de fabrication, mais encore et surtout sans que les interactions entre les électrons du faisceau pulsé et la matière thermoplastique ne compromettent l'utilisation ultérieure du récipient stérilisé.

Par comparaison avec un faisceau d'électrons pulsé selon le document FR-2.861.215, la durée d'émission d'une impulsion est très brève puisque cette durée est inférieure à 100 ns dans le cas de l'invention, par exemple de quelques nanosecondes, alors qu'elle s'exprime en microseconde (µs) avec un émetteur comme le canon à électrons à anode focalisante selon ce document.

Le faisceau d'électrons pulsé selon l'invention est par exemple obtenu au moyen d'une émission explosive d'électrons, encore parfois désignée par l'acronyme E.E.E pour « *Explosive Electron Emission »* en anglais.

La brièveté d'une durée d'émission du faisceau d'électrons pulsé de l'ordre de la nanoseconde conjuguée à une intensité de l'impulsion élevée s'exprimant en kilo-Ampère limite les interactions des électrons avec la matière thermoplastique tout en ayant une irradiation permettant une stérilisation efficace.

Les résultats obtenus avec l'invention sont particulièrement surprenants d'autant que ceux obtenus jusqu'alors, tant avec un faisceau d'électrons continu, qu'avec un faisceau d'électrons pulsé (comme celui produit avec un canon à électrons selon le document FR-2.861.215) dissuadaient l'homme du métier de poursuivre la stérilisation de l'intérieur d'un récipient au moyen d'un faisceau d'électrons.

Pour l'Homme du métier il n'est par conséquent pas possible d'une part de stériliser rapidement à travers sa paroi l'intérieur d'un récipient au moyen d'un faisceau d'électrons et, d'autre part, de le faire sans en altérer la matière thermoplastique.

L'utilisation d'un faisceau d'électrons va par conséquent à l'encontre des préjugés de l'Homme du métier. En effet pour l'Homme du métier, il n'est pas possible avec un faisceau d'électrons de stériliser l'intérieur d'un récipient en l'irradiant depuis l'extérieur à travers la paroi sans se heurter à des altérations rédhibitoires de la matière thermoplastique par le faisceau d'électrons, ainsi qu'à des durées d'irradiation pour obtenir une dose létale qui sont incompatibles avec une application industrielle.

Avantageusement, ledit faisceau d'électrons pulsé est émis avec, entre deux impulsions successives, un intervalle de temps (T) qui est supérieur à 3 ms, par exemple égal à 10 ms.

Un tel intervalle de temps participe avantageusement à limiter les interactions des électrons du faisceau avec la matière thermoplastique.

En l'absence d'un intervalle de temps substantiel par rapport à la durée d'émission d'une impulsion, l'irradiation obtenue avec le faisceau d'électrons pulsé diffère finalement peu de celle obtenue avec un faisceau d'électrons continu.

Par comparaison avec les irradiations réalisées avec les solutions de l'état de la technique, les micro-organismes sont de manière surprenante détruits avec une efficacité supérieure par un faisceau d'électrons pulsé conforme aux enseignements de l'invention.

Tout particulièrement, la destruction des micro-organismes est plus efficace lorsque l'irradiation est réalisée avec une succession d'impulsions très brèves, de forte intensité (i) et avantageusement un intervalle de temps (T) entre deux impulsions successives déterminé.

Avantageusement, le faisceau d'électrons pulsé irradie les micro-organismes en alternant de manière répétée des moments de « stress » des micro-organismes au cours desquelles ces derniers sont irradiés avec une forte intensité, avec un instant de répit entre deux « stress » successifs.

Avantageusement, ledit faisceau d'électrons pulsé présente une énergie, dite basse énergie, qui est inférieure à 500 KeV, de préférence supérieure à 400 KeV.

De préférence, ladite étape d'introduction du réflecteur est réalisée préalablement à l'étape d'irradiation. En variante, l'étape d'introduction du réflecteur est réalisée pendant l'étape d'irradiation, notamment de manière à réduire la durée totale de traitement par irradiation pour stériliser un récipient.

Avantageusement, le réflecteur est stérilisé par le faisceau d'électrons pulsé selon l'invention, la surface externe du réflecteur étant au moins irradiée par les électrons du faisceau lorsque le réflecteur s'étend axialement à l'intérieur du récipient.

Avantageusement, la mise en œuvre d'un tel procédé de stérilisation est compatible avec les cadences de fabrication de récipients et donc susceptible de recevoir une application industrielle en intégrant un dispositif de stérilisation au sein d'une installation de fabrication de récipients tels que des bouteilles en PET.

Avantageusement, la stérilisation du récipient selon le procédé de l'invention est effectuée en irradiant un récipient vide, de préférence juste avant de procéder à son remplissage.

Par comparaison avec un procédé de stérilisation de préformes par voie chimique selon le document WO-2006/136498 précité, l'invention permet de simplifier grandement la conception d'une installation de fabrication de récipients et d'en réduire les coûts d'exploitation.

La stérilisation du récipient final (et non de la préforme) permet avantageusement de s'affranchir de nombreux moyens jusqu'alors mis en œuvre dans une installation de fabrication de récipients à partir d'une préforme, les micro-organismes présents étant détruits lors de l'irradiation du récipient au moyen du faisceau d'électrons pulsé selon l'invention.

En effet en stérilisant le récipient et par comparaison toujours, il n'est notamment plus besoin de mettre en oeuvre des moyens spécifiques (tels que des systèmes d'insufflation, etc.) pour préserver la stérilité d'une préforme postérieurement à son traitement, c'est-à-dire au cours de son conditionnement thermique, de sa transformation par soufflage ou étirage-soufflage en récipient et cela jusqu'au remplissage et à la fermeture du récipient.

Avantageusement, le procédé de stérilisation selon l'invention permet de stériliser tant l'intérieur que l'extérieur du récipient.

Les dispositifs de traitement des préformes par irradiation au moyen d'un rayonnement UV sont par exemple susceptibles d'être supprimés au même titre que les systèmes d'insufflation et plus généralement de filtration de l'air participant à l'obtention d'un environnement de fabrication propre.

De préférence, le procédé de stérilisation selon l'invention est mis en œuvre dans l'installation de fabrication de récipients entre l'unité de moulage (ou souffleuse) et l'unité suivante, tel qu'une unité de remplissage.

L'invention propose encore un dispositif de stérilisation comportant au moins un émetteur d'un faisceau d'électrons pulsé et un réflecteur associé introduit axialement au moins en partie à l'intérieur dudit récipient pour réfléchir sélectivement tout ou partie dudit faisceau d'électrons pulsé émis par ledit émetteur depuis l'extérieur, à travers une paroi du récipient, pour irradier ledit récipient afin de stériliser au moins l'intérieur dudit récipient.

Avantageusement, ledit dispositif de stérilisation est destiné à la mise en œuvre du procédé décrit précédemment.

Selon d'autres caractéristiques du dispositif de stérilisation selon l'invention :
- le réflecteur est monté mobile axialement, relativement au récipient, entre au moins une première position dans laquelle le réflecteur s'étend à l'extérieur du récipient et une deuxième position dans laquelle le réflecteur, introduit par une ouverture que délimite un col du récipient, s'étend axialement au moins en partie à l'intérieur dudit récipient ;
- le réflecteur présente une réflectance qui varie axialement, ledit réflecteur comportant au moins une première partie présentant une réflectance et une deuxième partie présentant une réflectance qui est inférieure à la réflectance de la première partie ;
- la première partie du réflecteur présentant la réflectance et la deuxième partie du réflecteur présentant la réflectance sont respectivement réalisées dans des matériaux différents ;
- le réflecteur comporte au moins une partie spécifique présentant au moins une surface de réflexion qui ne s'étend pas dans un plan axial ;
   -- ladite au moins une partie spécifique de réflexion est située à l'extrémité axiale libre du réflecteur ;
   -- ladite au moins une partie spécifique de réflexion est formée par une bague s'étendant radialement en saillie vers l'extérieur ;
   -- ladite au moins une partie spécifique de réflexion comporte au moins une surface de réflexion tronconique ;
- le réflecteur comporte au moins une partie présentant une charge électrique déterminée, ladite charge étant négative pour obtenir un effet de répulsion des électrons ou positive pour obtenir un effet d'absorption des électrons ;
   -- le dispositif comporte des moyens d'entraînement en rotation du récipient pour entraîner en rotation sur lui-même ledit récipient relativement à l'émetteur du faisceau d'électrons pulsé.

D'autres caractéristiques et avantages de l'invention apparaîtront à la lecture de la description qui va suivre pour la compréhension de laquelle on se reportera aux dessins annexés dans lesquels :

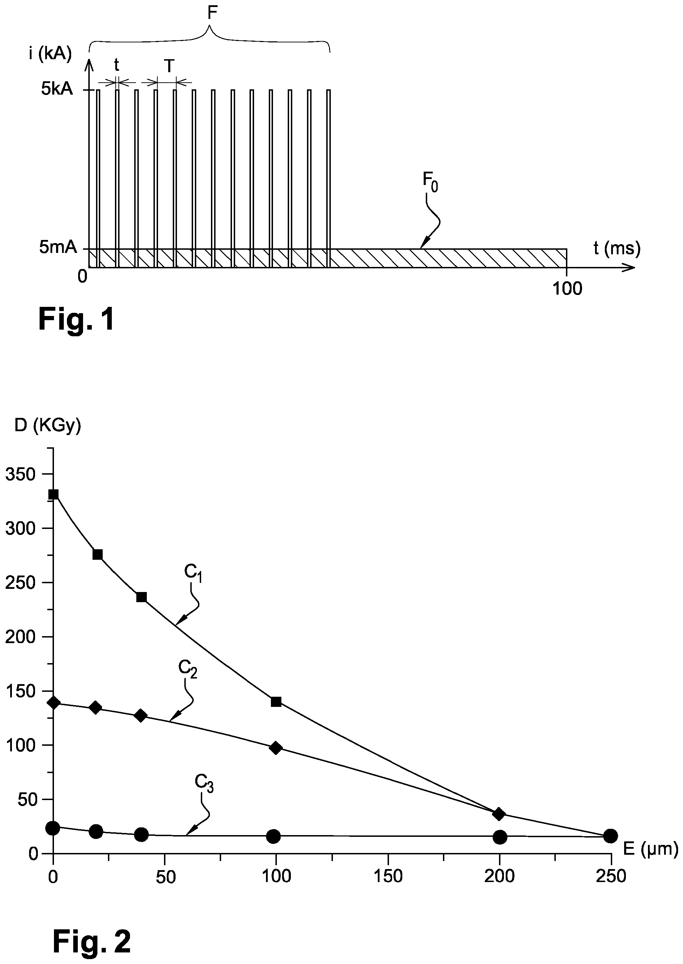

- la figure 1 est une représentation graphique qui représente en ordonnée l'intensité (i) exprimée en kilo-Ampère (kA) et en abscisse le temps exprimé en milliseconde (ms) et qui illustre respectivement un faisceau Fo d'électrons continu et un faisceau F d'électrons pulsé selon l'invention qui est formé par une série d'impulsions caractérisées par leur durée (t) d'émission, leur intensité (i) et la fréquence d'émission des impulsions avec un intervalle (T) entre deux impulsions ;
- la figure 2 est un graphique qui représente en ordonnée la dose (D) d'électrons reçus exprimée en kilo-Gray (kGy) et en abscisse l'épaisseur (E) exprimée en micromètre (µm) de la paroi d'un récipient en PET et qui illustre la dose déposée sur la surface extérieure du récipient et à travers la paroi pour obtenir une dose létale déterminée sur la surface interne du récipient, la courbe C1 correspondant à une irradiation du récipient avec un faisceau d'électrons continu, la courbe C2 avec un faisceau d'électrons pulsé présentant un niveau d'énergie de 250 KeV et la courbe C3 avec un faisceau d'électrons pulsé présentant un niveau d'énergie de 430 KeV ;

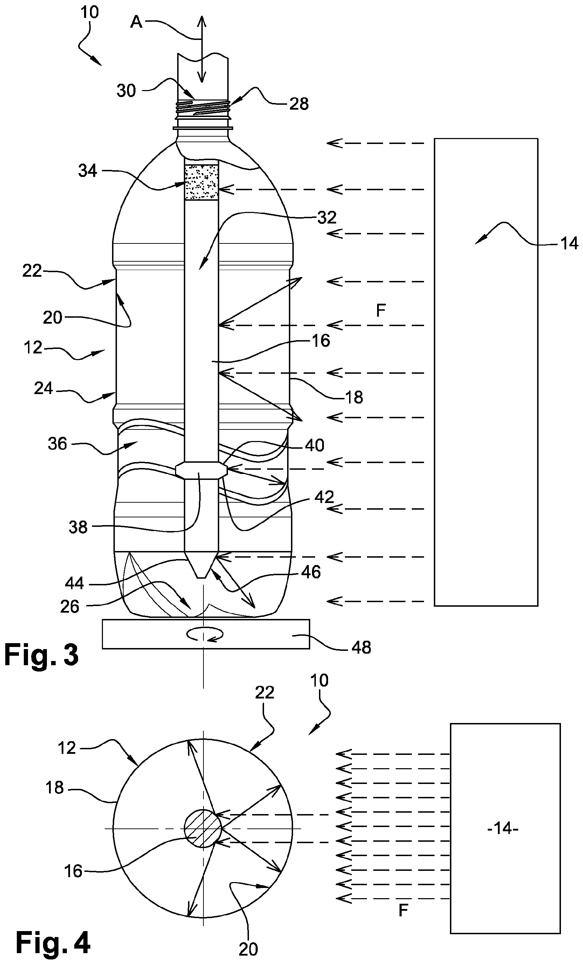

- la figure 3 est une vue de côté qui représente un exemple de réalisation d'un dispositif de stérilisation selon l'invention et qui illustre l'irradiation d'un récipient par le dispositif de stérilisation comportant un émetteur d'un faisceau d'électrons pulsé émis radialement depuis l'extérieur et qui est associé à un réflecteur introduit axialement à l'intérieur du récipient ;
- la figure 4 est une vue de dessus qui représente le récipient et le dispositif de stérilisation selon la figure 3 et qui illustre la stérilisation du récipient par le faisceau conformément à l'invention.

Le procédé de stérilisation de récipients en matière thermoplastique selon l'invention comporte au moins une étape d'irradiation consistant à irradier un récipient depuis l'extérieur au moyen d'un faisceau (F) d'électrons pulsé qui est formé d'une succession d'impulsions présentant chacune une durée (d) d'émission qui est inférieure à 100 ns et une intensité (i) qui est supérieure à 1 kA pour stériliser à travers une paroi du récipient l'intérieur dudit récipient.

Avantageusement, ledit faisceau (F) d'électrons pulsé est émis avec, entre deux impulsions successives, un intervalle de temps (T) qui est supérieur à 3 ms.

Avantageusement, ledit faisceau (F) d'électrons pulsé présente une énergie, dite basse énergie, qui est inférieure à 500 KeV.

De préférence, ledit faisceau (F) d'électrons pulsé présente une énergie supérieure à 400 KeV par exemple de l'ordre de 430 à 450 KeV.

En variante, ledit faisceau (F) d'électrons pulsé présente une énergie, dite basse énergie, inférieure à 500 KeV qui est par exemple égale à 250 KeV.

On a représenté sur le graphique de la figure 1, un faisceau (F) d'électrons pulsé selon l'invention qui est formé par une série d'impulsions, la durée (t) d'émission de chaque impulsion étant égale à 10 ns et avec une intensité de 5 kA.

Ledit faisceau (F) d'électrons pulsé de la figure 1 présente un niveau d'énergie de 250 KeV, soit une valeur inférieure à 500 KeV correspondant à une valeur de seuil généralement admise entre la « basse énergie » et la haute énergie.

De préférence, ledit faisceau (F) d'électrons pulsé représenté sur la figure 1 est émis avec, entre deux impulsions successives, un intervalle de temps (T) qui est égal à 10 ms.

A des fins de comparaison, on a également représenté sur la figure 1 un faisceau (F₀) d'électrons continu (hachuré) qui se distingue notamment du faisceau (F) d'électrons pulsé par l'absence de série d'impulsions entre chacune desquelles l'intensité (i) revient à une valeur nulle.

Le faisceau (F₀) d'électrons continu représenté présente une énergie 200 KeV, soit une basse énergie, et une intensité égale à 5 mA, la durée d'émission pour procéder à une stérilisation par irradiation étant de l'ordre d'au moins une seconde.

Par comparaison entre ces deux types de faisceau d'électrons, on constate que le faisceau d'électrons continu requiert une durée d'émission du faisceau pour irradier qui est plus longue et cela pour une moindre quantité d'électrons reçus.

En effet, le faisceau d'électrons pulsé consistant en la répétition d'une série d'impulsions très brèves permet d'irradier la surface à stériliser avec une quantité plus importante d'électrons, notamment en raison de l'intensité très supérieure de chaque impulsion du faisceau d'électrons pulsé par rapport à l'intensité du faisceau d'électrons continu.

L'intensité d'une impulsion égale à 5 kA est très nettement supérieure à celle de 5 mA du faisceau d'électrons continu.

Grâce à cette intensité de l'ordre du kA, le nombre d'électrons du faisceau (F) d'électrons pulsé traversant la paroi du récipient pour irradier les micro-organismes présents à l'intérieur du récipient va permettre de stériliser tant l'extérieur que l'intérieur du récipient, et cela sur toute sa hauteur soit axialement du col jusqu'au fond.

L'irradiation obtenue avec un faisceau (F) d'électrons pulsé est également efficace sur des parties du récipient présentant des surfaces complexes par exemple du fait du « design » du récipient.

Grâce à un faisceau d'électrons pulsé selon l'invention, une dose létale est appliquée sur la surface interne du récipient à stériliser et cela en transmettant une énergie moindre à la matière thermoplastique du récipient traversée par ce faisceau (F) d'électrons pulsé, ce qui limite avantageusement les risques d'altérations de la matière mais sans sacrifier à l'efficacité de la stérilisation.

L'irradiation des micro-organismes par un faisceau (F) d'électrons pulsé est plus efficace car les micro-organismes se protègent plus difficilement de la répétition des impulsions présentant les caractéristiques de durée (t) et d'intensité (i) du faisceau (F) d'électrons pulsé.

Avantageusement, la durée de traitement avec un faisceau (F) d'électrons pulsé est inférieure à celle qui serait nécessaire avec un faisceau (F₀) d'électrons continu pour obtenir une irradiation par une quantité équivalente d'électrons.

La figure 2 est une représentation graphique illustrant la dose (D) exprimée en kilo-Gray (kGy) en fonction de l'épaisseur (E) en micromètre (µm) de la paroi d'un récipient en PET, depuis la surface externe jusqu'à l'intérieur du récipient à stériliser.

La dose (D) en kilo-Gray (kGy) correspond à des Joules par kg (kilogramme), soit une énergie par unité de volume, qui correspondant à une dose cumulée illustre l'énergie cédée par les électrons et absorbée par le matériau du récipient.

La courbe C1 correspond à une irradiation avec un faisceau (F₀) d'électrons continu, la courbe C2 correspond à une irradiation avec un faisceau (F₁) d'électrons pulsé présentant un niveau d'énergie de 250 KeV et la courbe C3 correspond à une irradiation avec un faisceau (F₂) d'électrons pulsé présentant un niveau d'énergie de 430 KeV.

La valeur de 250 µm correspond à une valeur typique pour une paroi d'un récipient tel qu'une bouteille en PET.

La figure 2 représente la dose de rayonnement absorbée à travers une paroi de 250 µm de PET avec les différents faisceaux (F₀), (F₁) et (F₂) pour obtenir à l'intérieur du récipient une dose d'une valeur au moins égale à 14 kGy.

Tel qu'illustré sur la figure 2, l'énergie absorbée par le PET pour obtenir la dose létale recherchée d'au moins 14 kGy à une profondeur de 250 µm est bien inférieure lorsque le faisceau d'électrons est de type pulsé par rapport à un faisceau (F₀) d'électrons continu, et en comparant les deux faisceaux de type pulsé l'énergie absorbée est encore moindre avec le faisceau (F₂) d'énergie de 430 KeV qu'avec le faisceau (F₁) d'énergie de 250 KeV.

Un faisceau (F) tel que le faisceau (F₂) d'électrons pulsé présentant une énergie de 430 KeV permet d'obtenir une irradiation plus homogène du récipient à travers la paroi, de la surface extérieure et de la surface intérieure.

Avantageusement, l'énergie absorbée par le PET est moindre avec un tel faisceau (F₂) ce qui réduit les risques d'altération de la matière thermoplastique. Plus grande est l'énergie du faisceau, plus grande sera la quantité d'électrons traversant la paroi du récipient pour en irradier la surface intérieure.

Un faisceau (F₂) présentant une énergie de 430 KeV permet, par comparaison avec le faisceau (F₁) d'énergie de 250 KeV, de réduire sensiblement la durée totale d'irradiation ce qui est particulièrement avantageux pour une mise en œuvre dans une installation de fabrication de récipients.

De préférence, le faisceau (F) présente une énergie supérieure à 400 KeV.

Pour améliorer encore l'irradiation et raccourcir le temps de traitement, l'invention propose d'associer à l'émetteur un réflecteur destiné à être introduit axialement à l'intérieur du récipient par l'ouverture du col afin de réfléchir sélectivement le faisceau (F) d'électrons pulsé.

Avantageusement, le procédé comporte, préalablement à l'étape d'irradiation, une étape consistant à introduire axialement un réflecteur à l'intérieur du récipient à stériliser.

On a représenté aux figures 3 et 4, un exemple de réalisation d'un dispositif 10 de stérilisation d'un récipient 12 destiné à la mise en œuvre du procédé de stérilisation qui vient d'être décrit.

Dans la suite de la description, on utilisera par convention, de manière non limitative, l'orientation « axiale » en référence à l'axe principal du récipient et la direction de déplacement du réflecteur ainsi que l'orientation « radiale » qui est orthogonale à l'orientation « axiale ».

Le dispositif 10 de stérilisation de récipients 12 comporte au moins un émetteur 14 d'un faisceau (F) d'électrons pulsé et un réflecteur 16 associé.

Le réflecteur 16 est introduit axialement au moins en partie à l'intérieur dudit récipient 12 pour réfléchir sélectivement tout ou partie dudit faisceau (F) d'électrons pulsé émis par ledit émetteur 14 depuis l'extérieur, radialement à travers une paroi 18 du récipient, pour irradier ledit récipient 12.

Pour faciliter la représentation du faisceau par nature diffus (nuage électronique), le faisceau (F) d'électrons a été représenté sous la forme de flèche d'orientation radiale, toutefois une telle représentation n'est toutefois nullement limitative et les rayons du faisceau (F) n'étant pas nécessairement orthogonaux à la direction axiale.

L'irradiation du récipient 12 est plus particulièrement destinée à stériliser l'intérieur du récipient, c'est à dire la surface interne 20 du récipient qui sera ultérieurement en contact avec un produit notamment un liquide alimentaire tel que de l'eau, du lait, un jus, etc.

Toutefois, l'irradiation étant réalisée depuis l'extérieur du récipient 12 et à travers la paroi 18 va également en stériliser la surface externe 22 de sorte que le récipient 12 est stérilisé dans son ensemble par le faisceau (F) d'électrons pulsé.

Le récipient 12 représenté sur les figures 3 et 4 est donné uniquement à titre d'exemple, le récipient 12 comporte un corps 24 cylindrique s'étendant axialement entre un fond 26 et un col 28, ledit col 28 délimitant radialement une ouverture 30.

La paroi 18 présente une épaisseur (E) donnée de matière thermoplastique, par exemple du PET, et le terme « paroi » doit être compris au sens large pour l'ensemble du récipient 12, axialement depuis le fond 26 jusqu'au col 28 et le corps 24.

Le réflecteur 16 est monté mobile axialement, relativement au récipient 12, entre au moins une première position (non représentée) et une deuxième position représentée sur la figure 3.

La première position correspond à une position dans laquelle le réflecteur 16 s'étend à l'extérieur du récipient 12, totalement en dehors du récipient 12.

La première position est notamment occupée par le réflecteur 16 après la stérilisation d'un récipient 12 et en attendant la stérilisation du récipient 12 suivant.

La deuxième position correspond à une position dans laquelle le réflecteur 16, introduit par l'ouverture 30 que délimite le col 28 du récipient 12, s'étend axialement au moins en partie à l'intérieur dudit récipient 12.

De préférence, le réflecteur 16 est associé à des moyens d'entraînement, tels qu'un actionneur, qui est commandé pour déplacer axialement, suivant la flèche A représentée sur la figure 3, le réflecteur 16 relativement au récipient 12 occupant une position fixe.

En variante, le réflecteur 16 pourrait être fixe et le récipient 12 déplacé axialement relativement au réflecteur 16 pour introduire ce dernier à l'intérieur du récipient 12.

Le réflecteur 16 est réalisé sous la forme d'une tige axiale présentant un diamètre externe maximal qui est inférieur au diamètre interne du col du récipient 12 de manière à pouvoir être introduit axialement à l'intérieur dudit récipient, de préférence sans contact avec le col 28 notamment.

Avantageusement, le réflecteur 16 présente une réflectance qui varie axialement selon la partie du réflecteur 16 considérée.

Le réflecteur 16 comporte au moins une première partie 32 présentant une réflectance R1 et une deuxième partie 34 présentant un réflectance R2 qui est inférieure à la réflectance R1 de la première partie 32.

De préférence, la deuxième partie 34 du réflecteur 16 présentant la réflectance R2 est située axialement sur le réflecteur 16 pour se situer au niveau du col 28 et/ou de l'épaulement du récipient 12 lorsque le réflecteur 16 occupe ladite deuxième position.

Avantageusement, ladite au moins une deuxième partie 34 du réflecteur 16 présentant la réflectance R2 inférieure est déterminée en fonction du « design » du récipient 12, la ou les parties de moindre réflectance comme la deuxième partie 34 étant située axialement sur le réflecteur 16 pour se trouver radialement en vis à vis de la ou des partie(s) du récipient 12 plus proches radialement du réflecteur 16 telles que l'épaulement du récipient 12 s'étendant en dessous du col 28.

Le réflecteur 16 est avantageusement réalisé, en tout ou en partie, dans au moins un matériau présentant une masse atomique relative élevée, de préférence supérieure à 180, tel que le tantale (Ta) le tungstène (W), le platine (Pt) ou l'or (Au).

La première partie 32 du réflecteur 16 présentant la réflectance R1 et la deuxième partie 34 du réflecteur 16 présentant la réflectance R2 sont par exemple obtenues en utilisant des matériaux différents pour chacune.

Tel que représenté sur les figures 3 et 4, la surface externe réfléchissante du réflecteur 16 est formée en tout ou en partie par une surface cylindrique qui réfléchit avec une incidence donnée les électrons du faisceau F émis de manière pulsé par l'émetteur 14 radialement à travers la paroi 18 du récipient 12.

Le faisceau F d'électrons pulsé selon l'invention arrive orthogonalement à la surface cylindrique du réflecteur 16 avant d'être réfléchi avec une incidence donnée vers la surface interne 20 du récipient 12 à stériliser.

Cependant, un récipient 12 tel qu'une bouteille en PET présente généralement un « design » particulier et de ce fait une ou des zones, telle que la zone 36 spécifique ici en forme d'onde, ne présentant pas une surface cylindrique qui s'étende axialement parallèlement à celle du réflecteur 16 mais comportant des portions saillantes et/ou en creux.

Pour améliorer l'irradiation de telles zones spécifiques, le réflecteur 16 comporte au moins une partie 38 spécifique de manière à réfléchir le faisceau F d'électrons pulsé en direction d'au moins une zone 36 spécifique associée.

Avantageusement, ladite partie 38 spécifique du réflecteur 16 comporte au moins une surface de réflexion qui ne s'étend pas dans un plan axial.

Dans l'exemple, ladite au moins une surface de réflexion n'est pas parallèle à la surface interne de la paroi 18, ni orthogonale au faisceau F d'électrons émis radialement à travers la paroi 18 du récipient 12 par ledit émetteur 14

Dans l'exemple de réalisation, ladite partie 38 spécifique est constituée par au moins une bague s'étendant radialement en saillie par rapport au reste du réflecteur 16 et présentant en coupe axiale un profil en forme de « V » couché.

La partie 38 spécifique en forme de bague comporte une surface 40 supérieure de réflexion et une surface 42 inférieure de réflexion, respectivement tronconique.

Avantageusement, le réflecteur 16 comporte une autre une partie 34 spécifique de réflexion qui est située à l'extrémité axiale libre du réflecteur 16.

De préférence, ladite autre partie spécifique 44 de réflexion comporte au moins une surface 46 de réflexion tronconique destinée à réfléchir le faisceau F d'électrons en direction du fond 26, généralement de forme pétaloïde.

Avantageusement, le réflecteur 16 comporte au moins une partie présentant une charge électrique déterminée, ladite charge étant négative pour obtenir un effet de répulsion des électrons du faisceau F ou positive pour obtenir un effet d'absorption desdits électrons.

Une variation de la réflectance suivant la position axiale, d'une partie donnée du réflecteur par rapport à une autre, est susceptible d'être obtenue avec des parties présentant des charges électriques différentes.

De préférence, le réflecteur 16 est relié électriquement à la terre ou masse.

Avantageusement, le dispositif 10 comporte des moyens 48 d'entraînement en rotation du récipient 12 pour l'entraîner en rotation sur lui-même relativement à l'émetteur 14 du faisceau F d'électrons pulsé.

Le dispositif 10 de stérilisation qui vient d'être décrit constitue le ou l'un des postes de stérilisation d'une unité de stérilisation d'une installation de fabrication de récipients 12 en matière thermoplastique à partir de préformes chaudes.

Une telle unité de stérilisation de récipients 12 comportant au moins un dispositif 10 de stérilisation est agencée en aval de l'unité de moulage dans laquelle les préformes chaudes, par exemple préalablement conditionnées thermiquement dans un four, sont transformées en récipients 12 par soufflage ou par étirage-soufflage au moyen d'au moins un fluide sous pression.

Avantageusement, l'unité de stérilisation est agencée en amont d'une unité de remplissage des récipients 12 que comporte l'installation de fabrication de récipients 12 en matière thermoplastique.

Une installation de fabrication de récipients 12 en matière thermoplastique de ce type est connue de l'état de la technique et l'on se reportera par exemple au document WO-99/03667, donné toutefois à titre non limitatif.

## Revendications

1. Procédé de stérilisation d'un récipient (12) en matière thermoplastique et du type de récipients obtenus à partir d'une préforme chaude transformée par soufflage au moyen d'un fluide sous pression, le procédé comportant au moins une étape d'irradiation consistant à irradier le récipient (12) depuis l'extérieur au moyen d'un faisceau (F) d'électrons pour stériliser à travers une paroi (18) du récipient l'intérieur (20) dudit récipient (12), **caractérisé en ce que** le faisceau d'électron est un faisceau d'électrons pulsé qui est formé d'une succession d'impulsions présentant chacune une durée (d) d'émission qui est inférieure à 100 ns et une intensité (i) qui est supérieure à 1 kA, **en ce que** le procédé comporte une étape consistant à introduire axialement un réflecteur (16) à l'intérieur du récipient (12) à stériliser.

2. Procédé de stérilisation selon la revendication 1, **caractérisé en ce que** ledit faisceau (F) d'électrons pulsé est émis avec, entre deux impulsions successives, un intervalle de temps (T) qui est supérieur à 3 ms.

3. Procédé de stérilisation selon la revendication 1 ou 2, **caractérisé en ce que** ledit faisceau (F) d'électrons pulsé présente une énergie, dite basse énergie, qui est inférieure à 500 KeV.

4. Dispositif (10) de stérilisation pour la mise en œuvre du procédé de stérilisation selon l'une quelconque des revendications précédentes, comportant au moins un émetteur (14) d'un faisceau (F) d'électrons, **caractérisé en ce que** l'émetteur (14) est apte à émettre un faisceau (F) d'électrons pulsé et **en ce que** le dispositif (10) de stérilisation comprend un réflecteur (16) associé configuré pour être introduit axialement au moins en partie à l'intérieur dudit récipient (12) pour réfléchir sélectivement tout ou partie dudit faisceau (F) d'électrons pulsé émis par ledit émetteur (14) depuis l'extérieur, à travers une paroi (18) du récipient, pour irradier ledit récipient (12) afin de stériliser au moins l'intérieur (20) dudit récipient (12).

5. Dispositif de stérilisation selon la revendication 4, **caractérisé en ce que** le réflecteur (16) est monté mobile axialement, relativement au récipient (12), entre au moins une première position dans laquelle le réflecteur (16) s'étend à l'extérieur du récipient (12) et une deuxième position dans laquelle le réflecteur (12), introduit par une ouverture (30) que délimite un col (28) du récipient, s'étend axialement au moins en partie à l'intérieur dudit récipient (12).

6. Dispositif de stérilisation selon la revendication 4 ou 5, **caractérisé en ce que** le réflecteur (16) présente une réflectance qui varie axialement, ledit réflecteur (16) comportant au moins une première partie présentant une réflectance (R1) et une deuxième partie présentant un réflectance (R2) qui est inférieure à la réflectance (R1) de la première partie.

7. Dispositif de stérilisation selon la revendication 6, **caractérisé en ce que** la première partie du réflecteur (16) présentant la réflectance (R1) et la deuxième partie du réflecteur (16) présentant la réflectance (R2) sont respectivement réalisées dans des matériaux différents.

8. Dispositif de stérilisation selon l'une quelconque des revendications 4 à 7, **caractérisé en ce que** le réflecteur (16) comporte au moins une partie (38, 44) spécifique présentant au moins une surface (40, 42, 46) de réflexion qui ne s'étend pas dans un plan axial.

9. Dispositif de stérilisation selon l'une quelconque des revendications 4 à 8, **caractérisé en ce qu'**il comprend des moyens de faire varier la réflectance suivant la position axiale, d'une partie donnée du réflecteur par rapport à une partie reliée à la terre, lesquels moyens sont agencés pour que ladite partie du réflecteur (16) présente une charge électrique déterminée, ladite charge étant négative pour obtenir un effet de répulsion des électrons ou positive pour obtenir un effet d'absorption des électrons.

10. Système comprenant un dispositif (10) de stérilisation d'un récipient en matière thermoplastique selon l'une des revendications 4 à 9, et comprend ledit récipient, préalablement obtenu à partir d'une préforme chaude transformée par soufflage au moyen d'un fluide sous pression, le système étant agencé pour stériliser, à travers une paroi (18) du récipient, l'intérieur (20) dudit récipient (12).

## Patentansprüche

1. Verfahren zur Sterilisation eines Behälters (12) aus thermoplastischem Material und von der Art von Behältern, die aus einer heißen Vorform erhalten werden, die durch Blasen mittels eines unter Druck stehenden Fluids umgeformt wird, wobei das Verfahren zumindest einen Bestrahlungsschritt aufweist, der darin besteht, den Behälter (12) von außen mittels eines Elektronenstrahls (F) zu bestrahlen, um das Innere (20) des Behälters durch eine Wand (18) des Behälters (12) hindurch zu sterilisieren, **dadurch gekennzeichnet, dass** der Elektronenstrahl ein gepulster Elektronenstrahl ist, der aus einer Folge von Impulsen gebildet ist, die jeweils eine Emissionsdauer (d) von weniger als 100 ns und eine Intensität (i) von mehr als 1 kA aufweisen, und dass das Verfahren einen Schritt aufweist, der darin besteht, einen Reflektor (16) axial in das Innere des zu sterilisierenden Behälters (12) einzuführen.

2. Sterilisationsverfahren nach Anspruch 1, **dadurch gekennzeichnet, dass** der gepulste Elektronenstrahl (F) mit einem Zeitintervall (T) zwischen zwei aufeinanderfolgenden Impulsen von mehr als 3 ms emittiert wird.

3. Sterilisationsverfahren nach Anspruch 1 oder 2, **dadurch gekennzeichnet, dass** der gepulste Elektronenstrahl (F) eine als Niedrigenergie bezeichnete Energie von weniger als 500 KeV aufweist.

4. Sterilisationsvorrichtung (10) zur Durchführung des Sterilisationsverfahrens nach einem der vorangehenden Ansprüche, umfassend zumindest einen Sender (14) eines Elektronenstrahls (F), **dadurch gekennzeichnet, dass** der Sender (14) dazu in der Lage ist, einen gepulsten Elektronenstrahl (F) zu emittieren, und dass die Sterilisationsvorrichtung (10) einen zugehörigen Reflektor (16) umfasst, der konfiguriert ist, um zumindest teilweise axial in das Innere des Behälters (12) eingeführt zu werden, um den vom Sender (14) von außen durch eine Wand (18) des Behälters hindurch emittierten gepulsten Elektronenstrahl (F) wahlweise ganz oder teilweise zu reflektieren, um den Behälter (12) bestrahlen, um zumindest das Innere (20) des Behälters (12) zu sterilisieren.

5. Sterilisationsvorrichtung nach Anspruch 4, **dadurch gekennzeichnet, dass** der Reflektor (16) relativ zum Behälter (12) axial beweglich zwischen zumindest einer ersten Position, in der sich der Reflektor (16) außerhalb des Behälters (12) erstreckt, und einer zweiten Position, in der sich der Reflektor (12), der durch eine von einem Hals (28) des Behälters begrenzte Öffnung (30) eingeführt ist, axial zumindest teilweise im Inneren des Behälters (12) erstreckt, montiert ist.

6. Sterilisationsvorrichtung nach Anspruch 4 oder 5, **dadurch gekennzeichnet, dass** der Reflektor (16) einen axial variierenden Reflexionsgrad aufweist, wobei der Reflektor (16) zumindest einen ersten Teil mit einem Reflexionsgrad (R1) und einen zweiten Teil mit einem Reflexionsgrad (R2), der kleiner als der Reflexionsgrad (R1) des ersten Teils ist, aufweist.

7. Sterilisationsvorrichtung nach Anspruch 6, **dadurch gekennzeichnet, dass** der den Reflexionsgrad (R1) aufweisende erste Teil des Reflektors (16) und der den Reflexionsgrad (R2) aufweisende zweite Teil des Reflektors (16) jeweils aus unterschiedlichen Materialien hergestellt sind.

8. Sterilisationsvorrichtung nach einem der Ansprüche 4 bis 7, **dadurch gekennzeichnet, dass** der Reflektor (16) zumindest einen bestimmten Teil (38, 44) aufweist, der zumindest eine Reflexionsfläche (40, 42, 46) aufweist, die sich nicht in einer axialen Ebene erstreckt.

9. Sterilisationsvorrichtung nach einem der Ansprüche 4 bis 8, **dadurch gekennzeichnet, dass** sie Mittel zum Variieren des Reflexionsgrads in Abhängigkeit von der axialen Position eines gegebenen Teils des Reflektors relativ zu einem mit der Erde verbundenen Teil umfasst, wobei die Mittel so angeordnet sind, dass der Teil des Reflektors (16) eine bestimmte elektrische Ladung aufweist, wobei die Ladung negativ ist, um einen Elektronenabstoßungseffekt zu erzielen, oder positiv, um einen Elektronenabsorptionseffekt zu erzielen.

10. System, umfassend eine Vorrichtung (10) zur Sterilisation eines Behälters aus thermoplastischem Material nach einem der Ansprüche 4 und 9 und umfassend den Behälter, der zuvor aus einer heißen Vorform erhalten wurde, die durch Blasen mittels eines unter Druck stehenden Fluids umgeformt wurde, wobei das System angeordnet ist, um durch eine Wand (18) des Behälters hindurch das Innere (20) des Behälters (12) zu sterilisieren.

## Claims

1. Method for sterilizing a thermoplastic container (12) of the type of containers obtained from a hot preform converted by blow moulding by means of a pressurized fluid, the method comprising at least one irradiating step consisting in irradiating the container (12) from the outside by means of an electron beam (F) with a view to sterilizing, through a wall (18) of the container, the inside (20) of said container (12), **characterized in that** the electron beam is a pulsed electron beam that is formed from a succession of pulses each having an emission duration (d) that is shorter than 100 ns and an amplitude (i) that is higher than 1 kA, and **in that** the method comprises a step consisting in introducing axially a reflector (16) into the inside of the container (12) to be sterilized.

2. Sterilizing method according to Claim 1, **characterized in that** said pulsed electron beam (F) is emitted with, between two successive pulses, a time interval (T) that is longer than 3 ms.

3. Sterilizing method according to Claim 1 or 2, **characterized in that** said pulsed electron beam (F) has an energy, referred to as the low energy, that is lower than 500 keV.

4. Sterilizing device (10) for implementing the sterilizing method according to any one of the preceding claims, comprising at least one emitter (14) of an electron beam (F), **characterized in that** the emitter (14) is able to emit a pulsed electron beam (F) and **in that** the sterilizing device (10) comprises an associated reflector (16) configured to be axially introduced at least partially into the inside of said container (12) with a view to selectively reflecting all or some of said pulsed electron beam (F) emitted by said emitter (14) from the outside through a wall (18) of the container, with a view to irradiating said container (12) in order to sterilize at least the inside (20) of said container (12) .

5. Sterilizing device according to Claim 4, **characterized in that** the reflector (16) is mounted so as to be able to move axially, relatively to the container (12), between at least a first position in which the reflector (16) lies outside of the container (12) and a second position in which the reflector (12), introduced via an aperture (30) that delineates a neck (28) of the container, lies axially at least partially inside said container (12).

6. Sterilizing device according to Claim 4 or 5, **characterized in that** the reflector (16) has a reflectance that varies axially, said reflector (16) comprising at least a first portion having a reflectance (R1) and a second portion having a reflectance (R2) that is lower than the reflectance (R1) of the first portion.

7. Sterilizing device according to Claim 6, **characterized in that** the first portion of the reflector (16) having the reflectance (R1) and the second portion of the reflector (16) having the reflectance (R2) are respectively made of different materials.

8. Sterilizing device according to any one of Claims 4 to 7, **characterized in that** the reflector (16) comprises at least one specific portion (38, 44) having at least one reflecting surface (40, 42, 46) that does not lie in an axial plane.

9. Sterilizing device according to any one of Claims 4 to 8, **characterized in that** it comprises means for making the reflectance vary as a function of axial position, of a given portion of the reflector with respect to a portion connected to ground, which means are arranged so that said portion of the reflector (16) has a determined electric charge, said charge being negative with a view to obtaining an electron repulsion effect or positive with a view to obtaining an electron absorption effect.

10. System comprising a device (10) for sterilizing a thermoplastic container according to one of Claims 4 to 9, and comprising said container, which is obtained beforehand from a hot preform converted by blow moulding by means of a pressurized fluid, the system being arranged to sterilize, through a wall (18) of the container, the inside (20) of said container (12).
